(19) Europäisches Patentamt

European Patent Office

Office européen des brevets

(11) Veröffentlichungsnummer: **0 411 477 A1**

## (12) EUROPÄISCHE PATENTANMELDUNG

(21) Anmeldenummer: 90114360.2

(22) Anmeldetag: 26.07.90

(51) Int. Cl.⁵: **C11D 1/66**, A61K 7/16

(30) Priorität: 04.08.89 DE 3925858

(43) Veröffentlichungstag der Anmeldung:
06.02.91 Patentblatt 91/06

(84) Benannte Vertragsstaaten:
GR

(71) Anmelder: **Henkel Kommanditgesellschaft auf Aktien**
**Henkelstrasse 67**
**D-4000 Düsseldorf 13(DE)**

(72) Erfinder: **Hill, Karlheinz, Dr.**
**Am Hasenbusch 1**
**D-4006 Erkrath(DE)**
Erfinder: **Förg, Franz, Dr.**
**Rotdornweg 10**
**D-4018 Langenfeld(DE)**
Erfinder: **Körner, Hermann**
**Kamper Weg 292**
**D-4000 Düsseldorf(DE)**
Erfinder: **Penninger, Josef, Dr.**
**Mozartstrasse 64**
**D-4010 Hilden(DE)**

(54) **Pulverförmige Zubereitungen oberflächenaktiver Alkylglykoside.**

㊵ Pulverförmige Zubereitungen oberflächenaktiver Alkylglycoside, enthalten 5 - 65 Gew.-% eines oberflächenaktiven Alkylglykosids und 35 - 95 Gew.-% eines inerten anorganischen Trägers. Sie werden dadurch hergestellt, daß man das bei der technischen Herstellung der Alkylglykoside anfallende Rohprodukt mit Wasser und einem anorganischen, partikelförmigen Träger, z. B. Kieselsäure, Kreide oder Natriumchlorid, mischt und trocknet. Bevorzugte pulverförmige Zubereitungen enthalten 20 - 50 Gew.-% des Alkylglykosids und 50-80 Gew.-% eines inerten anorganischen Trägers.

EP 0 411 477 A1

# PULVERFÖRMIGE ZUBEREITUNGEN OBERFLÄCHENAKTIVER ALKYLGLYKOSIDE

Die Erfindung betrifft pulverförmige Zubereitungen oberflächenaktiver Alkylglykoside mit guter Rieselfähigkeit sowie ein Verfahren zu ihrer Herstellung durch Trocknung einer wäßrigen Mischung von Alkylglykosid und inerten partikelförmigen Trägermaterialien.

Oberflächenaktive Alkylglykoside im Sinne der vorliegenden Erfindung sind die Reaktionsprodukte aus Zucker und aliphatischen primären Alkoholen mit vorzugsweise 8 - 22 Kohlenstoffatomen. Als Zuckerkomponenten, die im folgenden als Glykosen bezeichnet werden, kommen bevorzugt Glucose, daneben aber auch Fructose, Mannose, Galactose, Talose, Gulose, Allose, Altrose, Idose, Arabinose, Xylose, Lyxose, Ribose und Gemische in Frage.

Bevorzugt wegen der leichten Zugänglichkeit und der guten anwendungstechnischen Eigenschaften sind die Acetalisierungsprodukte der Glucose mit Fettalkoholen, die z. B. aus natürlichen Fetten und Ölen nach bekannten Verfahren erhältlich sind, d. h. mit linearen, primären, gesättigten und ungesättigten Alkoholen mit 8 - 22 Kohlenstoffatomen. Die Erfindung bezieht sich daher bevorzugt auf diese Typen der Alkylglykoside, im folgenden auch Alkylglucoside genannt.

Bei der Herstellung der Alkylglykoside nach den verschiedenen bekannten Verfahren z. B. nach US-3.839.318 oder EP 132 046 A1, werden diese mit unterschiedlichen Restmengen an nicht umgesetzten Ausgangsprodukten erhalten, von welchen insbesondere der Fettalkohol sich bei verschiedenen Anwendungen störend bemerkbar macht. Die rohen Acetalisierungsprodukte stellen feste, erstarrte Schmelzen dar, die sich bei Normaltemperatur nicht gießen und verteilen lassen.

Nach einem besonders bevorzugten Herstellungsverfahren wird Glucose mit Fettalkohol acetalisiert, wobei kontinuierlich Wasser bei reduziertem Druck aus dem Gemisch abdestilliert wird. Bei diesem Herstellungsverfahren, welches in der deutschen Patentanmeldungen P 38 33 780.0 näher beschrieben ist, fällt das rohe Alkylglucosid als erstarrte Schmelze an, die circa 63 Gew.-% Fettalkyl-Monoglucosid, 15 Gew.-% Fettalkyl-Diglucosid, 6 Gew.-% Fettalkyl-Triglucosid, 3 Gew.-% Alkyl-Tetraglucosid, 6 Gew.-% Polyglucose und 2 - 4 Gew.-% Rest-Fettalkohol enthält. Versuche, diese Schmelze durch Sprühkonfektionierung, durch Verschuppen oder Granulieren in ein bei Normaltemperatur rieselfähiges, partikelförmiges Produkt umzuwandeln, führten zu klebrigen und feuchtigkeitsempfindlichen Materialien. Der im Produkt noch enthaltene unumgesetzte Rest-Fettalkohol macht sich außerdem bei der Verwendung der Alkylglucoside in Mund- und Zahnreinigungsmitteln geschmacklich unangenehm bemerkbar. Mund- und Zahnpflegemittel stellen aber ein bevorzugtes Anwendungsgebiet für die Schleimhaut-verträglichen, oberflächenaktiven Alkylglykoside dar.

Es bestand daher die Aufgabe, oberflächenaktive Alkylglykoside in eine schütt- und rieselfähige Zubereitungsform zu überführen, die sich problemlos lagern, fördern, verteilen und in andere Produkte, insbesondere in Mund- und Zahnpflegemittel, problemlos einarbeiten läßt.

Gegenstand der Erfindung sind pulverförmige Zubereitungen oberflächenaktiver Alkylglykoside, die dadurch gekennzeichnet sind, daß sie 5 - 65 Gew.-% eines oberflächenaktiven Alkylglykosids und 35 - 95 Gew.-% eines inerten Trägers enthalten, und die durch Trocknung einer gießfähigen wäßrigen Mischung von rohem Alkylglykosid und Träger enthalten werden.

Unter einem rohen Alkylglykosid wird das nach dem bekannten Herstellverfahren anfallende Rohprodukt mit den verfahrensbedingten Nebenbestandteilen, insbesondere das nach dem oben beschriebenen Acetalisierungsverfahren erhältliche Reaktionsgemisch mit 2 - 5 Gew.-% an nicht umgesetzten Fettalkoholen verstanden.

Als Träger eignen sich alle chemisch und physiologisch inerten, in Wasser weitgehend neutral, allenfalls schwach basisch, reagierenden und nach dem Trocknen partikelförmigen anorganischen Trägermaterialien. Besonders bevorzugt sind solche Stoffe, die beim Einsatz in Mund- und Zahnpflegemittel, z. B. in Zahnpasten, nicht störend sind, z. B. weil sie bereits in anderer Funktion in solchen Zubereitungen erwünscht sind. Solche geeigneten Träger sind z. B. die bekannten Poliermittel-Komponenten, wie Kreide, Kieselsäuren, Dicalciumphosphat, Calciumpyrophosphat, unlösliches Hatrium-Metaphosphat, Aluminiumoxid und Aluminiumoxidhydrate. Es eignen sich aber auch neutrale und physiologisch akzeptable wasserlösliche Salze wie z. B. Kochsalz oder Natriumsulfat oder Gemische von zwei oder mehreren dieser Träger. Weitere geeignete inerte Träger sind Alumosilikate, z. B. Schichtsilikate, Talkum, Zeolithe, Magnesium-aluminiumsilikat (Veegum[(R)]), Calciumsulfat, Magnesiumcarbonat oder Magnesiumoxid.

Besonders bevorzugt als Träger sind Kieselsäuren, die durch ihre hohe innere Oberfläche bereits in geringen Mengen ab etwa 0,5 Gewichtsteilen pro 1 Gewichtsteil Alkylpolyglukosid eine gute rieselfähige Zubereitung ergeben. Bei anderen Trägerstoffen, z. B. bei Kreide und bei Kochsalz und Natriumsulfat sollte das Gewichtsverhältnis von Träger zu Alkylglykosid wenigstens 1 : 1, besser noch 1 - 4 : 1 betragen, um anwendungstechnisch günstige und ausreichend lagerstabile Zubereitungen zu bekommen. Bevorzugte erfindungsgemäße Zubereitungen enthalten daher 20 - 50 Gew.-% eines oberflächenaktiven Alkylglykosids und 50 - 80 Gew.-% eines inerten anorganischen Trägers.

Bevorzugt werden solche feinteiligen abrasiven Kieselsäuren als Träger verwendet, die auch bereits als Poliermittel für Zahnpasten in Gebrauch sind. Dies sind z. B. die Fällungskieselsäuren, wie sie aus DE 31 14 492 und DE 31 14 493 oder aus DE 24 46 038 bekannt und im Handel erhältlich sind, die wasserhaltigen Gelkieselsäuren, wie sie aus DE 27 04 504 und DE 28 53 647 bekannt sind und die Xerogelkieselsäuren, wie sie z. B. aus US 35 38 230 bekannt sind. Es können aber auch verdickende Kieselsäuren und pyrogene Kieselsäuren, z. B. Aerosil, verwendet oder mitverwendet werden, wenn besonders niedrige Schüttdichten angestrebt werden.

Durch die Wahl der Art und Menge des Trägers und durch Kombinationen verschiedener Träger lassen sich die physikalischen und anwendungstechnischen Eigenschaften der erfindungsgemäßen pulverförmigen Zubereitungen in weiten Bereichen steuern. Für solche Zubereitungen, die als Zusatz zu Zahnpasten geeignet sein sollen und als Träger einen wasserunlöslichen Abrasivstoff enthalten, sollte die Partikelgröße des Trägers überwiegend unter 50 $\mu$ liegen und im Mittel bevorzugt 0,1 - 10 $\mu$ betragen.

Die Herstellung der erfindungsgemäßen Zubereitungen erfolgt dadurch, daß man 5 - 65 Gewichtsteile eines bei der technischen Herstellung anfallenden rohen Alkylglykosids mit 35 - 95 Gewichtsteilen eines anorganischen Trägers und so viel Wasser mischt, daß eine bei einer Temperatur unterhalb + 80 °C noch fließfähige Mischung entsteht, und dieser Mischung das Wasser durch Verdampfung so weit entzieht, daß ein pulverförmiges, gut rieselfähiges Produkt entsteht. Man kann in diese Mischung auch die 30- bis 60 prozentige wäßrige Paste des Alkylglucosids einsetzen, die nach der Bleichung des rohen Alkylglucosids nach dem Verfahren gemäß Deutscher Patentanmeldung P 38 33 780.0 erhalten wird. Es hat sich als günstig erwiesen, zur Herstellung dieser Mischung etwa 1,5 - 4 Gewichtsteile Wasser auf einen Gewichtsteil Feststoff einzusetzen. Diese Mischung stellt in der Regel eine bei Normaltemperatur wenig fließbare Paste (Slurry) dar, die beim Erwärmen auf Temperaturen bis 80 °C gut pumpfähig und versprühbar wird. In der Paste sind die wasserlöslichen Bestandteile weitgehend gelöst bzw. gequollen, die wasserunlöslichen Komponenten sind darin stabil dispergiert.

Der Entzug des Wassers aus dem Gemisch, d. h. die Trocknung der Paste bzw. des Slurry kann auf beliebige Weise erfolgen, z. B. auf Walzen- oder Bandtrocknern, in einem Wirbelbett aus fertiger Zubereitung oder durch Versprühen in einen Heißluftstrom. Das letztgenannte Verfahren, die sogenannte Sprühtrocknung, ist die bevorzugte Ausführungsform für die Trocknung der erfindungsgemäßen Zubereitungen. Der Entzug des Wassers erfolgt so weit, daß ein pulverförmiges, gut rieselfähiges Produkt entsteht. Dies ist in den meisten Fällen bei Wassergehalten von weniger als 1 Gew.-% der Fall. In Fällen, in denen der Träger einen Teil des Wassers als Kristallwasser bindet, bezieht sich dieser empfohlene Rest-Wassergehalt auf das freie, nicht im Kristallgitter gebundene Wasser. In Fällen, in welchen es bei der Trocknung zur Bildung von größeren Agglomeraten kommt, z. B. bei der Trocknung auf Walzen- und Bandtrocknern, kann es erforderlich sein, diese Agglomerate in einem nachgeschalteten Mahlprozeß zu desintegrieren. Bei Entfernung des Wassers durch Sprühtrocknung ist eine nachträgliche Desintegration der erfindungsgemäßen Zubereitungen nicht erforderlich. Zur Verwendung in Zahnpasten sollten die erfindungsgemäßen Zubereitungen keine Agglomerate mit Durchmessern über 200 $\mu$ enthalten, während die Primär-Korngröße im Mittel nicht über 50 $\mu$ liegen sollte.

Die erfindungsgemäßen Zubereitungen sind gut rieselfähig und behalten diese Rieselfähigkeit bei Lagerung auch in feuchter Luft bei. Ein weiterer Vorteil der pulverförmigen Zubereitungen liegt darin, daß durch das erfindungsgemäße Herstellverfahren der Gehalt an Rest-Fettalkohol stark verringert wird. Die Zubereitungen können daher auch zur Herstellung von Mund- und Zahnpflegemitteln verwendet werden, ohne daß es zu geschmacklichen Beeinträchtigungen kommt.

Die folgenden Beispiele sollen den Gegenstand der Erfindung näher erläutern ohne ihn hierauf zu beschränken.

### Beispiele

Aus den folgenden Rohstoffen wurden Mischungen gemäß Tabelle hergestellt. Die Mischung Nr. 1 wurde in einem Trockenturm unter folgenden Sprühbedingungen versprüht:

Trocknungsluft: Menge: 600 m³/h
Eintrittstemperatur: 170 °C
Austrittstemperatur: 155 °C
Sprühdüse: Zweistoffdüse, 1mm Ø

Pastendruck: 4,0 bar

Zerstäubungsluft: 4,0 bar

Dabei wurde ein rieselfähiges, feines weißes Pulver mit hervorragender Fließfähigkeit (Schüttwinkel 24,7 °) und einer Restfeuchte von 0,24 Gew.-% $H_2O$ erhalten. Die Partikelgröße lag zwischen 1 und 180 $\mu$ mit Maxima bei 8 $\mu$ (Primärkorn) und 100 $\mu$ (Agglomerat). Die Mischungen Nr. 2 - 7 wurden im Laboratorium im Rotationsdampfer getrocknet. Dabei wurden ebenfalls gut rieselfähige, nicht klebende Pulver erhalten.

Rohstoffe:

A) Alkylglucosid aus einem linearen $C_{12}/C_{14}$-Fettalkohol,

hergestellt nach Patentanmeldung P 38 33 780, Beispiel 3, enthaltend:

3,0 Gew.-% restlichen Fettalkohol $C_{12}/C_{14}$

62,8 Gew.-% Alkylmonoglucosid

15,4 Gew.-% Alkyldiglucosid

5,8 Gew.-% Alkyltriglucosid

2,5 Gew.-% Alkyltetraglucosid

1,1 Gew.-% Alkylpentaglucosid

0,2 Gew.-% Alkylhexaglucosid

6,0 Gew.-% Polyglucose

unter 1,0 Gew.-% Glucose

unter 2,0 Gew.-% Salze

B) Natriumchlorid

C) Natriumsulfat, wasserfrei

D) Kreide (Schäfer Kreide N)

E) Fällungskieselsäure, Type Sident 12 SPLS,

Degussa

F) Fällungskieselsäure, Type Sipernat 22 LS 22 SL, Degussa

Tabelle I

| Mischung Nr. | 1 | 2 | 3 | 4 | 5 | 6 | 7 |
|---|---|---|---|---|---|---|---|
| Alkylglucosid S | 10 | 15 | 10 | 5 | 15 | 10 | 10 |
| Sident 12 SPLS | 20 | 15 | - | - | - | - | - |
| Sipernat 22 LS | - | - | 10 | 10 | - | - | - |
| Schäfer Kreide N | - | - | - | - | 30 | - | - |
| NaCl | - | - | - | - | - | 20 | - |
| $Na_2SO_4$ | - | - | - | - | - | - | 20 |
| Wasser | 70 | 70 | 80 | 85 | 65 | 70 | 70 |

Die Gehalte an restlichem Fettalkohol $C_{12}/C_{14}$ und der Rest-Wassergehalt lagen in allen durch Sprühtrocknung erhaltenen Pulvern unter 1 Gew.-%.

**Ansprüche**

1. Pulverförmige Zubereitungen oberflächenaktiver Alkylglykoside, dadurch gekennzeichnet, daß sie 5 - 65 Gew.-% eines oberflächenaktiven Alkylglycosids und 35 - 95 Gew.-% eines inerten anorganischen Trägers enthalten.

2. Pulverförmige Zubereitungen nach Patentanspruch 1, dadurch gekennzeichnet, daß sie 20 - 50 Gew.-% eines oberflächenaktiven Alkylglykosids und 50 - 80 Gew.-% eines inerten anorganischen Trägers enthalten.

3. Pulverförmige Zubereitungen nach einem der Patentansprüche 1 oder 2, dadurch gekennzeichnet, daß sie durch Trocknung einer gießfähigen, wäßrigen Mischung von rohem Alkylglykosid und Träger erhalten werden.

4. Pulverförmige Zubereitungen nach einem der Ansprüche 1 - 3, dadurch gekennzeichnet, daß als inerter anorganischer Träger Kreide, Kieselsäuren, Dicalciumphosphat, Calciumpyrophosphat, wasserunlösliches Natriummetaphosphat, Aluminiumoxid, Aluminiumoxid-hydrat, Kochsalz, Natriumsulfat oder ein Gemisch aus zwei oder mehreren dieser Stoffe enthalten ist.

5. Verfahren zur Herstellung pulverförmiger Zubereitungen von oberflächenaktiven Alkylglykosiden, dadurch gekennzeichnet, daß man 10 - 60 Gewichtsteile eines bei der technischen Herstellung anfallenden rohen Alkylglykosids mit 50 - 90 Gewichtsteilen eines inerten anorganischen Trägers und mit so viel Wasser mischt, daß eine bei einer Temperatur unterhalb 80 °C noch fließfähige Mischung entsteht, und dieser Mischung das Wasser durch Verdampfung so weit entzieht, daß ein pulverförmiges, rieselfähiges Produkt entsteht.

6. Verfahren nach Patentanspruch 5, dadurch gekennzeichnet, daß man zur Herstellung der Mischung 1,5 - 4 Gewichtsteile Wasser auf einen Gewichtsteil Feststoff einsetzt.

Europäisches
Patentamt

**EUROPÄISCHER
RECHERCHENBERICHT**

Nummer der Anmeldung

**EP 90 11 4360**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | WO-A-8 702 053 (A.E. STALEY MANUFACTURING CO.) * Patentansprüche; Seite 2, Zeilen 1-12; Seite 8, Zeile 21 - Seite 12, Zeile 24; Seite 13, Zeilen 5-30; Seite 14, Zeile 19 -Seite 17, Zeile 19; Beispiel 1 * <br> − − − | 1-6 | C 11 D 1/66 <br> A 61 K 7/16 |
| X | US-A-4 536 319 (PAYNE) * Patentansprüche; Spalte 2, Zeile 53 - Spalte 3, Zeile 61; Beispiel 1 * <br> − − − | 1-6 | |
| X | EP-A-0 304 627 (HENKEL) * Patentansprüche; Beispiel 2.3 * <br> − − − − − | 1 | |

| RECHERCHIERTE SACHGEBIETE (Int. Cl.5) |
|---|
| C 11 D <br> A 61 K |

Der vorliegende Recherchenbericht wurde für alle Patentansprüche erstellt

| Recherchenort | Abschlußdatum der Recherche | Prüfer |
|---|---|---|
| Den Haag | 21 November 90 | WILLEKENS G.E.J. |